# EUROPEAN PATENT SPECIFICATION

(11) **EP 2 988 832 B1**
(45) Date of publication and mention of the grant of the patent: **05.09.2018**
(21) Application number: 14726841.1
(22) Date of filing: 24.04.2014
(51) Int. Cl.: A61Q 9/02, B26B 21/44, A61K 8/86

(54) **LUBRICATING SKIN ENGAGING MEMBER**
GLEITENDES HAUTKONTAKTELEMENT
ÉLÉMENT LUBRIFIANT ENTRANT EN CONTACT AVEC LA PEAU

(30) Priority: 24.04.2013 US 201361815468 P
(43) Date of publication of application: 02.03.2016
(73) Proprietor: The Gillette Company LLC, Boston, MA 02127 (US)
(72) Inventor: STEPHENS, Alison, Fiona, Egham Surrey TW20 9NW (GB); JONES, Neil , John, Egham Surrey TW20 9NW (GB); BRADFORD, Valerie, Jean, Boston, Massachusetts 02127 (US); BAXTER, Elaine, Alice Marie, Egham Surrey TW20 9NW (GB)
(74) Representative: Töpert, Verena Clarita
(86) International application number: PCT/US2014/035228
(87) International publication number: WO 2014/176391

(56) References cited:
- US-A- 5 454 164
- US-A- 5 653 971
- US-A1- 2012 090 180
- US-A1- 2012 093 897
- US-A1- 2012 094 003
- US-A1- 2013 042 482

## Description

### FIELD OF THE INVENTION

The present invention concerns skin engaging members, especially those for use on hair removal cartridges or devices, and the cartridges or devices themselves.

### BACKGROUND OF THE INVENTION

The use of shaving aids on razor blades to provide lubrication benefits during the shave is known. *See e.g.,* U.S. Patents 7,121,754; 6,298,558; 5,711,076; 5,134,775; 6,301,785 and U.S. Patent Publ. Nos. 2009/0223057, 2006/0225285. Such shaving aids typically comprise a water-insoluble matrix material to provide structural integrity and a water-soluble polymer such as polyethylene oxide (polyox) in order to provide the lubrication during the shave once the water-soluble polymer enters solution with water present during shaving. More recently, however, some shaving aids in the form of skin engaging members have been produced that comprise a container in which a shaving aid (especially e.g. polyox) is held. *See e.g.,* U.S. Patent Publ. No. 2011/0099815, and PCT Patent Publ. Nos. 2011/047221, 2011/047222, 2011/049892 and 2011/050130. Since the application of polyox as a shaving lubricant, little development has been made in the field, though polyox is not without its limitations. For example, the use of polyox with low molecular weight offers limited lubrication, and while improved lubrication may be seen when using polyox with higher molecular weights, this impacts other aspects of the aqueous solution typically formed in-use, for example the resultant viscosity in aqueous solution may also increase, leading to negatively perceived attributes, for example concerning the feeling of the shave for the user, particularly in respect of the lubricant. US5454164 describes a shaving aid comprising a matrix of a water insoluble polymer and an effective amount of a skin lubricating water soluble polymer dispersed in the matrix and a compatibiliser. US5653971 describes a shaving aid comprising a skin soothing agent comprised within a matrix of water insoluble and water soluble polymers. Accordingly, there remains a need for technologies that can break this paradigm in order to offer improved lubrication benefits, ideally without negative impact to consumer perception.

### SUMMARY OF THE INVENTION

According to a first aspect, a skin engaging member use on a hair removal device is provided, said skin engaging member comprising a carrier having a skin contacting surface, said skin contacting surface forming at least one orifice; and a lubricating material releasably engaged with said carrier, said at least one orifice exposing at least a portion of said lubricating material; wherein the lubricating material comprises a copolymer of polyethylene oxide and polypropylene oxide wherein said copolymer has an average molecular weight of from 12000 to 13000.

According to a second aspect, a hair removal cartridge, having a first end and an opposing second end is provided; the hair removal cartridge comprising: at least one hair removal member positioned between said first end and said second end; and at least one skin engaging member according to the first aspect.

According to a third aspect, a hair removal device is provided, the hair removal device comprising a hair removal cartridge according to the second aspect; and a handle permanently or removably attached to the hair removal cartridge.

### BRIEF DESCRIPTION OF THE DRAWINGS

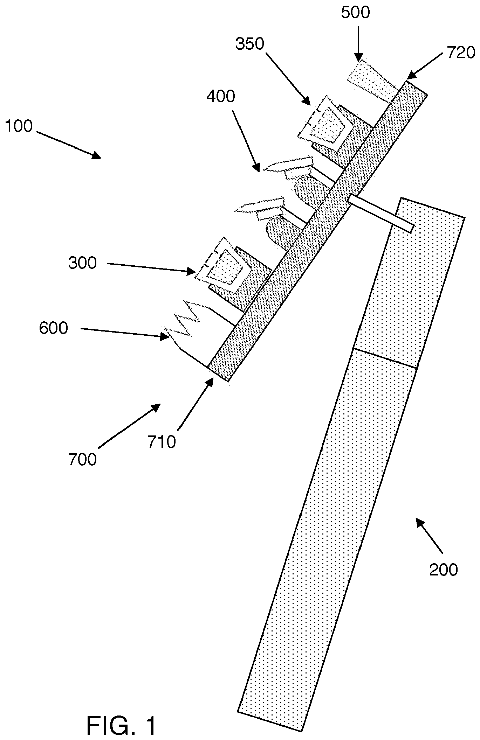

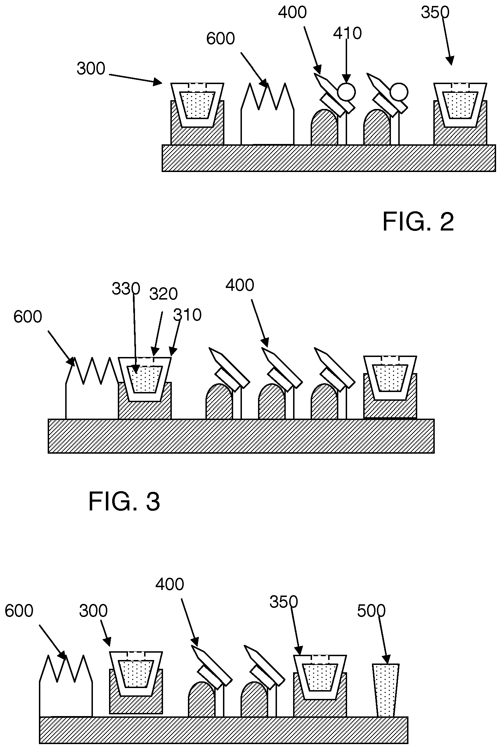

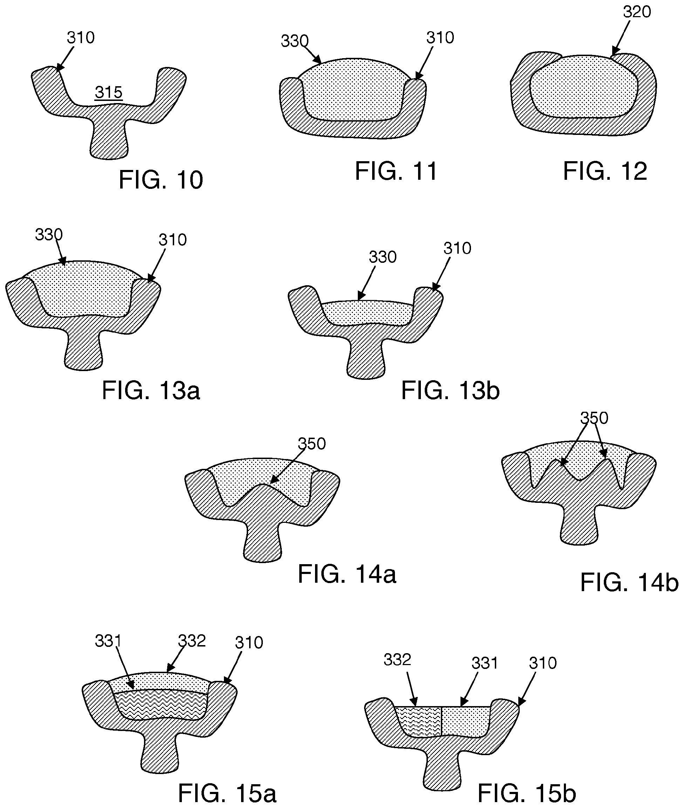

FIG. 1 is a side view of a hair removal device in accordance with the present invention. FIGs. 2 and 3 are cross sectional side views of additional hair removal cartridges in accordance with the present invention. FIGs. 4 - 9 are top planar views of various hair removal cartridges in accordance with the present invention. FIGs. 10, 11, 12, 13a, 13b, 14a, 14b, 15a, and 15b are side cross sectional views of various other skin engaging members in accordance with the present invention. FIG. 16 is a chart showing coefficient of friction values for various comparative examples.

### DETAILED DESCRIPTION OF THE INVENTION

The skin engaging members of the present invention typically allow for the loading of various lubricating materials onto a hair removal device for delivery during the hair removal process. The details of the skin engaging member and its location on any hair removal device according to the invention will be disclosed herein with figures showing exemplary embodiments which can include various elements of the present invention. Those of skill in the art will understand that various combinations of elements described in the specification and disclosed in the figures can be used in accordance with the present invention. The skin engaging member is intended to be suitable for use during the hair removal process, such as shaving, and the lubricating material(s) can therefore include various known compounds commonly used for topical application in personal care.

The skin-engaging member (also termed a shaving aid member or skin-engaging shaving aid member) comprises a lubricant, or lubricating material. The lubricating material can be in various forms, as well as mixtures/combinations thereof, as will be described below.

### I. Copolymer of Polyethylene Oxide and Polypropylene Oxide

The lubricating material comprises a copolymer of polyethylene oxide (PEO) and polypropylene oxide (PPO) to improve the lubrication properties of the skin engaging member.

The PEO/PPO copolymer may have any average molecular weight. Advantageously, the PEO/PPO copolymer has an average molecular weight of from 12,000 to 13,000 and even more preferably still from 12,250 to 12,750. Without wishing to be bound by theory, the inclusion of a PEO/PPO copolymer of sufficient molecular weight is thought to further improve the lubrication properties of the skin engaging member in aqueous conditions, especially in combination with a further water soluble polymer (particularly polyethylene oxide), and thus prevent an undesirable feeling in use.

The PEO/PPO copolymer may be of any arrangement but is advantageously a block copolymer, for example a di-block, tri-block, multi-block, radial-block or random-block copolymer. Preferably, the PEO/PPO copolymer is a tri-block copolymer, more preferably a tri-block copolymer having the sequence: PEO-PPO-PEO. Such tri-block copolymers of PEO and PPO are commercially available under tradenames such as Pluracare from BASF and Pluronic from Sigma-Aldrich.

The PEO/PPO copolymer may have any weight ratio of PEO to PPO (i.e. of ethylene oxide repeat units to propylene oxide repeat units), for example anywhere from 1000:1 to 1:1000 or from 100:1 to 1:100. Advantageously, the weight ratio is selected to improve the solubility properties of the PEO/PPO copolymer in a system comprising a water-soluble polymer (especially polyethylene oxide) and water, and so may be from 10:1 to 1:10, preferably from 1:1 to 1:7 (or any ratio in which the weight of PPO is greater than or equal to the weight of PEO), more preferably from 1:2 to 1:5, even more preferably from 1:2.5 to 1:4 and even more preferably still from 1:2.5 to 1:3.

An alternative way of describing the solubility properties of the PEO/PPO copolymer is through the well known hydrophilic-lipophilic balance (HLB). The PEO/PPO copolymer may have an HLB of from 0 to 50, but advantageously will have an HLB in the range of from 1 to 30, more preferably from 3% to 25%, even more preferably from 4% to 20% and even more preferably still from 5% to 10% by weight of the lubricating material or by weight of the skin engaging member, in order to provide an improved balance between any further water soluble polymer providing lubrication and the PEO/PPO copolymer to address the problems associated with the water soluble polymer. The remainder of the lubricating material may be entirely other water soluble polymer(s) (e.g. partially or entirely polyethylene oxide), or a mixture of further ingredients.

### II. Water Soluble Polymer

The lubricating material may also comprise a further water soluble polymer, typically intended to provide lubrication in-use.

Examples of water soluble polymers include polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and a mixtures thereof. In some embodiments, said water soluble polymer is selected from the group consisting of polyethylene oxide, polyethylene glycol, and mixtures thereof.

The water-soluble polymer will preferably comprise at least about 50%, more preferably at least about 60%, by weight of the skin engaging member, up to about 99%, (or up to about 90% of the lubricating material). For example, the water-soluble polymer may be present at an amount of at least about 50%, preferably from about 50% to about 99.9%, more preferably from about 60% to about 95% (e.g. from about 90% to about 95%) and even more preferably from about 70% to about 90% by weight of the lubricating material. Not all of the water-soluble polymer needs to meet the average molecular weight requirement, for example a blend of two or more grades of polyethylene oxide could be used wherein at least one, but less than all, of the grades meets the average molecular weight attribute, but the total amount of polyethylene oxide is within one of the ranges above. Alternatively, the average molecular weight for the entirety of the water-soluble polymer (especially polyethylene oxide) may fall within the desired average molecular weight range as well as the total amount of water-soluble polymer (especially polyethylene oxide) being according to one or more of the ranges above.

The more preferred water soluble polymers are the polyethylene oxides generally known as POLYOX (available from Union Carbide Corporation) or ALKOX (available from Meisei Chemical Works, Kyoto, Japan). The water soluble polymer, (especially these polyethylene oxides), will preferably have average molecular weights of at least about 5,000, at least about 20,000, at least about 50,000, at least about 100,000 or from about 100,000 to 6 million, preferably about 300,000 to 5 million. A particularly preferred polyethylene oxide comprises a blend of about 40% to 80% of polyethylene oxide having an average mol.wt. of about 5 million (e.g. POLYOX COAGULANT) and about 60% to 20% of polyethylene oxide having an average mol.wt. of about 300,000 (e.g. POLYOX WSR-N-750). The polyethylene oxide blend may also advantageously contain up to about 10% (for example about 5%) by weight of a low mol.wt. (i.e. MW<10,000) polyethylene glycol such as PEG-100.

### III. Water Insoluble Polymer

The lubricating material can further comprise a water-insoluble polymer, e.g. in which the other components of the lubricating material are dispersed, which may be referred to as a water-insoluble matrix, and may be helpful to improve the flow of the lubricating material when molten, e.g. if molding the lubricating material into the carrier. Desirably, the water insoluble polymer may be present at a level of from about 0.01% to about 50%, preferably from about 0.1% to about 30%, more preferably from about 0.5% to about 20% and even more preferably from about 1% to about 10% by weight of the lubricating material.

Suitable water-insoluble polymers which can be used include polyethylene (PE), polypropylene, polystyrene (PS), butadiene-styrene copolymer (e.g. medium and high impact polystyrene), polyacetal, acrylonitrile-butadiene-styrene copolymer, ethylene vinyl acetate copolymer, polyurethane, and blends thereof such as polypropylene/polystyrene blend or polystyrene/impact polystyrene blend.

One preferred water-insoluble polymer is polystyrene, preferably a general purpose polystyrene, such as NOVA C2345A, or a high impact polystyrene (HIPS) (i.e. polystyrene-butadiene), such as BASF 495F KG21. The lubricating material or any portion thereof may contain a sufficient quantity of water-insoluble polymer to provide additional mechanical strength, both during production and use.

### IV. Further Optional Ingredients

In some embodiments, the lubricating material comprises any other ingredients commonly found in commercially available shaving aid members or skin engaging members, such as those used on razor cartridges by Gillette, Schick or BIC. Non-limiting examples of such skin engaging members include those disclosed in U.S. 6301785, 6442839, 6298558, 6302785, and U.S Patent Pubs 2008/060201, and 2009/0223057.

The skin engaging member or lubricating material may therefore contain other conventional shaving aid ingredients, such as low mol.wt. water-soluble release enhancing agents such as polyethylene glycol (MW<10,000, e.g., 1-10% by weight PEG-100), water-swellable release enhancing agents such as cross-linked polyacrylics (e.g., 2-7% by weight), colorants, skin care actives, surfactants, soaps (including interrupted soaps), antioxidants, preservatives, emollients, lipids, oils, waxes, fats, cooling agents (especially non-volatile cooling agents), essential oils, beard softeners, astringents, medicinal agents, plasticizers, additional lubricants, depilatories/keratolytic materials, tackifiers, skin-soothing agents, fragrances, compatibilisers, anti-inflammatory agents, antipruritic/counterirritant materials etc.

Portions that contain a colorant can be designed to release the colorant (e.g., by leaching or abrasion), and thereby cause the skin engaging member (or a portion thereof) to change color during shaving, preferably in response to wear of the colored portion, so as to provide an indication to the user that the skin engaging member and/or the hair removal cartridge (e.g. razor cartridge) has reached the end of its effective life or the end of its optimum performance. A portion may contain, for example, between about 0.1% and about 5.0% (preferably between about 0.5% and 3%) colorant by weight.

In some embodiments, the lubricating material further comprises from about 0.5% to about 50%, preferably from about 1% to about 20%, polycaprolactone (preferably mol.wt. of 30,000 to 60,000). See US 6,302,785.

In some embodiments, the skin engaging member further comprises another shaving aid ingredient, for example selected from the group consisting of polyvinyl pyrrolidone, polyacrylamide, hydroxypropyl cellulose, polyvinyl imidazoline, polyethylene glycol, poly vinyl alcohol, polyhydroxyethylmethacrylate, silicone copolymers, sucrose stearate, vitamin E, panthenol, aloe, polyethylene glycol, silicone oil, Teflon® polytetrafluoroethylene powders (manufactured by DuPont), menthol, camphor, eugenol, eucalyptol, safrol and methyl salicylate; tackifiers such as Hercules Regalrez 1094 and 1126, cyclodextrins, inclusion complexes of skin-soothing agents with cyclodextrins; antimicrobial/keratolytic materials such as Resorcinol; anti-inflammatory agents such as Candilla wax and glycyrrhetinic acid; astringents such as zinc sulfate; surfactants such as iconol materials; compatibilizers such as styrene-b-EO copolymers; mineral oil, polycaprolactone (PCL), and combinations thereof.

### V. Skin Engaging Member

The skin engaging member forms at least one orifice for dispensing the lubricating material onto skin during use, for example the skin engaging member may comprise a carrier into which at least one orifice is formed. The carrier would in such a case typically have a receiving region for receiving the lubricating material.

The orifice may be of any shape and may, for example, have a cross sectional area of from about 0.0005 to about 0.25 square inches. Small orifices can also be provided with cross sectional area of from about 0.005 to about 0.05 square inches, or from about 0.01 to about 0.025 square inches. Larger orifices can have cross sectional areas of from about 0.05 to about 0.25 square inches, or from about 0.1 to about 0.2 square inches. Combinations of small and large orifices can also be provided on the same skin engaging member, or on separate skin engaging members on the same cartridge, depending on the desired dispense rate and amount of exposure of the lubricating material to water.

The skin engaging member will typically have a skin engaging surface which has a surface area while the at least one orifice (i.e. the sum for all orifices if a plurality are present) has a cross sectional area such that the surface area and cross sectional area are in a ratio of from about 50:1 to about 1:1, or about 25:1 to about 2:1, or about 10:1 to about 3:1. An individual orifice may also have a greatest lateral distance, varying depending on the number of orifices used, for example from about 1% to about 99% of the greatest lateral distance of the hair removal cartridge or a portion of the skin-engaging member corresponding to the exposed length of any hair removal members, or from about 2% to about 95%, or from about 3% to about 90%, or from about 5% to about 80%, or from about 10% to about 75%, or from about 15% to about 50%. Examples of suitable carriers include the sheaths disclosed in U.S. Patent No. 6,298,558 or 7,581,318. The one or more orifices may be arranged so as to substantially or entirely cover an area or length of the skin-engaging member, or a portion of the skin-engaging member corresponding to the exposed length of any hair removal members, i.e. the length available for hair removal, e.g. not covered as clips. For example, a plurality of orifices may be arranged in an array that spans substantially or entirely the exposed length of any hair removal members, or of the greatest lateral distance of the hair removal cartridge, in order that lubricating material may be provided across the area being shaved. Alternatively, the one or more orifices may be arranged so as to cover an area or length of the skin-engaging member or a portion of the skin-engaging member corresponding to the exposed length of any hair removal members in accordance with any of the percentage ranges or values described for the individual orifices above.

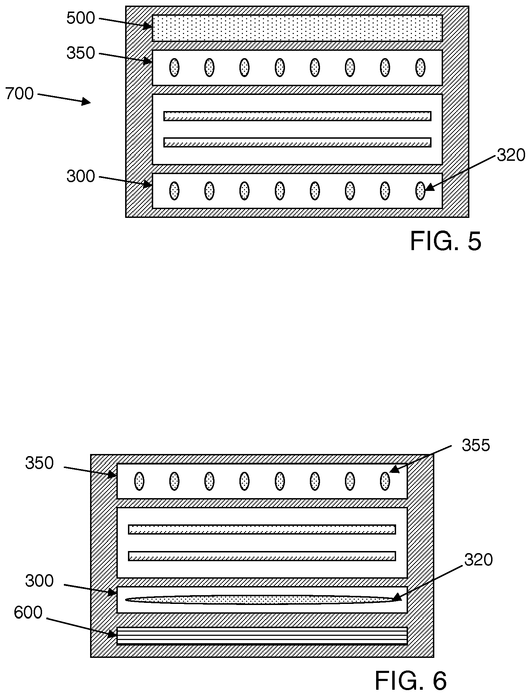

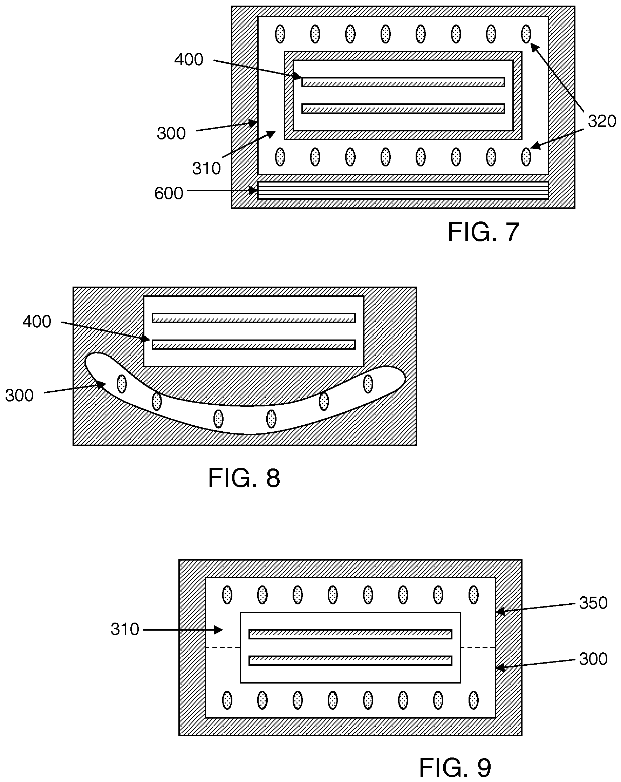

In some embodiments, at least a portion of said skin engaging member is not a straight line, for example the portion of said skin engaging member which is not a straight line can be angled or curvilinear. Curvilinear as defined herein means that at least a portion of the structure is curved such that it does not form a straight line. Where at least two skin engaging members are provided, they can also be positioned relative to one another such that they do not form a straight line. In one orientation, such as shown in FIG. 5, they can be parallel to each other. In another orientation, such as in FIG. 9, each skin engaging member includes a portion which does not form a straight line.

In some embodiments, the curved or angled nature of the skin engaging member is such that it forms at least a partial ring. A partial ring, as defined herein, means that the structure has at least two curved or angled sections which are concave to form an inner region. The partial ring can also include a curved or angled portion which are positioned convex to said inner region. One or more of said skin engaging members may also be positioned relative to one another to form a full ring. The ring can be formed by a single skin engaging member but two or more can be touching at or about their terminal ends, or even overlapping, to form such a ring.

The skin engaging member (or the carrier thereof) can have smooth curve sections as it forms the corners around the edges of the at least one elongated edge, or it can form sharp edges, e.g. which are affixed onto one another via melt bonding or adhesives. Where a non-linear section (curved, angled, partially twisted, etc) of the carrier is needed, the lubricating material can be formed integral with the carrier (formed at the same time), or they can be formed separately then later the lubricating material can be transferred into the carrier. The skin engaging member may comprise the carrier and the lubricating material be formed initially as a linear article and any non-linear deformations are created before the skin engaging member hardens and takes its form. The carrier (e.g. without any lubricating material contained therein) may also be formed and non-linear deformations then created, or the carrier can be molded or extruded having non-linear portions. The lubricating material can then be added, e.g. into the receiving region of the carrier. These and other ways of making the skin engaging member are also possible and will be described hereafter.

The carrier of the skin engaging member, i.e. the physical structure of the skin engaging member absent the lubricating material, can be formed of a variety of materials. The carrier may, for example, be non-water soluble such that it does not degrade or dissolve during normal use or can be partially water-soluble to allow for increased release of the lubricating material. A partially water-soluble carrier is preferably less soluble than the lubricating material and will preferably wear more slowly. In one embodiment, the carrier can be made of a shave aid or lubrication strip material as described above. Without intending to be bound by theory, it is believed that having a carrier which dissolves and/or wears more slowly than the lubricating material will allow for increased exposure of the lubricating material contained therein over time. Increasing exposure of the lubricating material over time will allow for maintained and/or increased release of the lubricating material even as it begins to wear down. This can be particularly desirable where the lubricating material is contained within the carrier.

The carrier typically has sufficient mechanical strength and rigidity to provide adequate mechanical strength to the entire skin engaging member, both as initially produced and after a significant amount of water soluble material has been leached out of the skin engaging member, or a further reinforcing member could be utilised. In some embodiments, the carrier comprises a base and one or more side walls, forming a receiving region, or channel, onto or into which the lubricating material is placed. The carrier may also form one or more retaining members within said receiving region extending away from said base. The retaining member may also be elongated and stretch across at least about 20% to about 100%, or from about 35% to about 75%, or about 50%, of the length of the carrier. A plurality of retaining members may also be used, for example in a linear or non-linear alignment throughout the receiving region, and can be spaced equidistantly or not. The retaining member(s) may have a height of from about 0.05 cm to about 1 cm, or from about 0.1 cm to about 0.5 cm. In other words, one or more (e.g. all) of the retaining members may have a height of from about 10% to about 100%, or from about 30% to about 60%, of the height of at least one of said side walls.

The side walls may or may not be the same height (as measured extending away from the base of the carrier). At least one of side walls can have a height of about 0.1 cm to about 1 cm, preferably from about 0.2 cm to about 0.4 cm. The pair of side walls can be biased away from each other as the walls extend away from said base, or they can be biased towards each other. One or both ends of the carrier can be enclosed, e.g. as described in US 7,581,318.

The carrier may be made of a water-insoluble polymer, particularly a thermoplastic resin. Thermoplastic resins are those materials which can be extruded or molded into a shape and are resilient under normal environmental conditions such as contact with water, even up to normal household hot water temperatures (for example up to 125 °C); normal wear and tear by consumers during use; device assembly and shipping, etc. Thermoplastic resins suitable for use in the carrier include polystyrene, high impact polystyrene (polystyrene-butadiene), polypropylene, filled polypropylene, polyethylene, nylon ethylene vinyl acetate, and blends such as 70% nylon/30% polyethylene oxide, 60% polystyrene/40% polyethylene oxide butadiene styrene copolymer, polyacetal, acrylonitrile-butadiene styrene copolymer, and mixtures thereof. The preferred resins are high impact polystyrene, polystyrene, ethylene vinyl acetate (EVA), and mixtures thereof.

For example, at least about 35%, at least about 50%, at least about 60%, at least about 70%, at least about 80%, at least about 90%, at least about 95%, at least about 99%, or 100% of the carrier may be water-insoluble polymer, or may be thermoplastic resin, or may be any of the polymers in the paragraph above, by weight of the carrier.

The carrier may also be made of shaving aid ingredients as described above. This can be particularly useful if the carrier is intended to be wearable and/or dissolvable throughout the usage life of the device and/or skin engaging member. Further, by providing a carrier made of a shave aid composition, both parts of the skin engaging member provide hair removal benefits during use.

The carrier can also be made of a solid polymeric composition, such as a mixture of water-soluble and water-insoluble polymers, for example similar to an additional lubrication material described below. Without intending to be bound by theory, this can be particularly desirable where it is desirable for the entire skin engaging member to be erodible or at least partially water soluble. In one embodiment, said water-insoluble polymer is present at a level of at least about 35% by weight of said carrier, or at least about 50%, or at least about 75%, or at least about 90%. The water-soluble polymer can be present as the remainder. Optionally, the carrier can include additives such as lubricants or plasticizers, fillers such as CaCO3, and colorants such as TiO2.

A wear indicating effect may be produced in the skin-engaging member, for example when the carrier and the lubricating material are made of disparately colored materials (e.g. white coloured sheath and blue coloured core). The lubricating material leaches out of the skin engaging member through use so that, with sufficient use, a differently coloured region within the lubricating material leaches out. Accordingly, a different layer within the lubricating material that is a different colour could be revealed, or a portion of the carrier may be revealed. By examining the orifices along the skin engaging surface, the user is provided with an indication that the shaving unit and/or skin engaging surface have reached their effective life. By way of example, the lubricating material may comprise a polyethylene oxide/polystyrene blend which is colored with Indigotine, FD&C #2 dye, and the carrier may comprise nylon and/or polystyrene which has been colored white.

### VI. Razor Cartridge

According to some embodiments of the invention, a hair removal cartridge (e.g. a razor cartridge or shaving head) is provided, comprising a housing, at least one hair removal member within the housing and at least one skin engaging member according to the invention as described hereinabove. The skin engaging member, (e.g. via the carrier), may be affixed to the cartridge by adhesive such as Loctite Super Bonder 499, by mechanical locking mechanism, by thermal welds or by a combination thereof.

Advantageously, the majority (i.e. at least 50%), or least 75%, or up to 100% of the outer periphery of the at least one hair removal member may be surrounded by one or more carriers (for example one skin engaging member forward of the hair removal members and one aft of the hair removal members). A single carrier may be used to surround at least a portion of said one or more hair removal members, or multiple carriers and/or various compositions within the same carrier may be used to surround at least a portion of said one or more hair removal members. The "periphery" of the hair removal members, as used herein, means the outer periphery of the structure upon which the hair removal member(s) are present. For example where a multi-blade razor is used, the periphery would be a ring around the outer edge of the box of blades.

The at least one skin engaging member may at least partially surround at least two sides of at least one hair removal member, preferably forming a partial ring, or even more preferably fully surrounding the entire periphery of the at least one hair removal member. As explained above, the at least one skin engaging member can be formed of a single skin engaging member or can be two or more skin engaging members.

The skin engaging member or members can form a 270 degree ring around the entire perimeter of the at least one elongated edge, or even a 360 degree ring. The carrier need not be in contact or be immediately adjacent to the elongated edge but by providing a ring around the elongated edge, the carrier is able to ensure that the lubricating material is deposited onto skin prior to and immediately following any contact of the elongated edge to skin.

The razor cartridge comprises one or more elongated edges or hair removal members (e.g. blades) usually positioned between a first and second end, said one or more elongated edges comprising a tip extending towards said first end. For example, U.S. Patent 7,168,173 generally describes a Fusion® razor that is commercially available from The Gillette Company and which includes a razor cartridge with multiple blades. Additionally, the hair removal cartridge, or razor cartridge, may include a guard as well as a skin engaging member. A variety of razor cartridges can be used in accordance with the present invention. Nonlimiting examples of suitable razor cartridges, with and without fins, guards, and/or shave aids, include those marketed by The Gillette Company under the Fusion®, Venus® product lines as well as those disclosed in U.S. Patent Nos. 7,197,825, 6,449,849, 6,442,839, 6,301,785, 6,298,558; 6,161,288, and U.S. Patent Publ. 2008/060201. Those of skill in the art will understand that the present skin engaging member can be used with any currently marketed system or disposable razor, including those having 2, 3, 4, 5 or more blades.

A hair removal member is a structure responsible for cutting, pulling or shearing off the hair from the skin. For example, the hair removal member may be one or more blades; a scraping edge which can be used after a depilatory composition is applied onto the skin and hair or a plurality of tweezer members which can be used for epilation (pulling hairs out of the follicle).

In some embodiments, said at least one skin engaging member is located on the portion of the cartridge that contacts skin during the shaving process, forward, between and/or aft of the hair removal member(s). A feature "forward" of the one or more hair removal members, for example, is positioned so that the surface to be treated with by the shaving device encounters the feature before it encounters the hair removal members. A feature "aft" of a hair removal member is positioned so that the surface to be treated by the shaving device encounters the feature after it encounters the hair removal member. Where more than one skin engaging member is provided on the shaving device, they can be the same (identical) or different, in terms of physical shape/structure and/or chemical composition.

In some embodiments, the cartridge comprises a guard comprising at least one elongated flexible protrusion to engage a user's skin. The at least one flexible protrusion may comprise flexible fins generally parallel to the one or more elongated edges (hair removal members). Said at least one flexible protrusion may additionally or alternatively comprise flexible fins comprising at least one portion which is not generally parallel to said one or more elongated edges. Non-limiting examples of suitable guards include those used in current razor blades and include those disclosed in U.S. Patent Nos. 7,607,230 and 7,024,776; (disclosing elastomeric / flexible fin bars); 2008/0034590 (disclosing curved guard fins); 2009/0049695A1 (disclosing an elastomeric guard having guard forming at least one passage extending between an upper surface and a lower surface). In some embodiments, said skin engaging member is positioned on the cartridge aft of the guard and forward of the hair removal members. In another embodiment, the skin engaging member is positioned on the cartridge forward of the guard. This embodiment can be particularly useful to deliver the lubricating material prior to contact with the guard.

The cartridge may further comprise an additional lubricating material (such as a known or commercially available shave aid or lubrication strip) to provide further lubrication to the hair removal experience. This additional lubricating material typically comprises a water insoluble polymer and a water soluble polymer. Such a shave aid or lubrication strip can be positioned within the ring of the skin engaging member according to the invention or outside the skin engaging member ring. Non-limiting examples of known lubricating materials suitable for use herein include shave aids and lubrication strips as described in: U.S. Patent Nos. 7,069,658, 6,944,952, 6,594,904, 6,302,785, 6,182,365, D424,745, 6,185,822, 6,298,558 and 5,113,585, and 2009/0223057.

### VII. Hair Removal Device

According to some embodiments of the invention, a hair removal device (e.g. a razor) is provided, which generally comprises a hair removal cartridge (e.g. a razor cartridge) according to the invention as described hereinabove, and a handle (or grip portion) permanently or removably attached to the cartridge. The hair removal device can be manual or power driven and can be used for wet and/or dry application. The hair removal cartridge may be replaceable and/or pivotally connected to the handle (e.g. via a cartridge connecting structure) and in turn or independently (e.g. permanently fixed) to a handle. In some embodiments, the cartridge connecting structure includes at least one arm to releasably engage the hair removal cartridge.

### VIII. Details on Figures

FIG. 1 is a side view of a hair removal device (100) in accordance with at least one embodiment of the present invention. The device comprises a hair removal cartridge (700) having a first end (710) and a second end (720), said cartridge being operably connected to a handle (200). In this example the hair removal cartridge includes two elongated edges (e.g. blades) (400), and a skin engaging member (300) positioned forward of said two elongated edges, and a skin engaging member (350) positioned aft of said two elongated edges. Further, a guard (400) is provided forward of said skin engaging member. An optional shave aid (500) is provided aft of said elongated edges.

FIG. 2 is a cross sectional side view of another hair removal cartridge in accordance with at least one embodiment of the present invention. Two elongated edges (400) are shown, which can be razor blades, having intrablade guards (410). In this example the first skin engaging member (300) is positioned forward of elongated edges and forward of the guard, wherein the guard is positioned between the elongated edges and the skin engaging member. Further shown here is a second skin engaging member (350), positioned aft of the elongated edges. The first and the second skin engaging members are preferably part of the same skin engaging member but they can also be different and separate structural elements.

FIG. 3 shows more than two elongated edges provided on the hair removal cartridge, e.g. three, four, or five elongated edges can be included. Also shown in this figure is the carrier (310) forming an orifice (320) to allow the lubricating material (330) to be dispensed during use onto skin. In this figure, the guard is positioned forward of the first skin engaging member. FIG. 4 shows the guard (600) positioned forward but not adjacent to the first skin engaging member (300) and a conventional solid polymeric shaving aid (500) positioned aft of the second skin engaging member (350).

FIG. 5 is a top planar view of a hair removal cartridge similar to that shown in FIG. 4, but without a guard. FIG. 6 is a top planar view of a hair removal cartridge where the first skin engaging member forms an orifice (320) having a greatest lateral distance which is at least a 50% of the greatest lateral distance of the hair removal cartridge. FIG. 6 also shows a second skin engaging member positioned aft of the elongated edges with smaller orifices (355). Further a guard (600) is positioned forward of the first skin engaging member.

FIG. 7 shows a top planar view of a hair removal cartridge in accordance with the present invention, wherein the skin engaging member at least partially encircles the elongated edges. Here the skin engaging member is shown forming a 360 ring around the elongated edges but a partial ring can also be within the scope of the present invention. In FIG. 7, the orifices do not extend around the periphery of the hair removal cartridge, but orifices can also be provided along the sides of the elongated edges if desired. The orifices may be equally spaced apart. A guard (600) with fins is also optionally provided forward of the skin engaging member and hair removal members.

FIG. 8 shows a top planar view of another hair removal cartridge with a skin engaging member (300) in accordance with the present invention. This skin engaging member has at least one portion which is curvilinear. This skin engaging member is positioned forward of the elongated edge (400) but it can also be provided aft of said edge. Further, two or more of such curved skin engaging members can be used to form an at least partial ring around the elongated edges.

FIG. 9 shows two skin engaging members (300) and (350) being provided wherein they can be formed of the same carrier, or separate carriers which appear to be connected when viewed from a top view. The lubricating materials can be the same or they can be different and tailored to deliver various lubricating materials based on their position on the hair removal cartridge.

FIGs. 10 - 15b are various side cross sectional views of skin engaging members in accordance with the present invention. FIG. 10 shows the carrier (310) forming a receiving region (315) into which lubricating material can be placed. FIGs. 11 and 12 show two additional side cross sectional view of skin engaging members where the side walls of the carrier can be shaped to form an orifice (320). FIGs. 13a and 13b show the same carrier (310) where the lubricating material (330) has been worn down from use. FIGs. 14a and 14b show two skin engaging members where the carrier forms at least one retaining member (350) extending away from the base of the carrier. FIGs. 15a and 15b show two skin engaging members with a first lubricating material (331) and a second lubricating material (332) forming discrete layers within the carrier (310). The layers can be adjacent or they can be separated by a retaining member if so desired. The layered lubricating materials shown in FIG. 15a can be created by repeated slot coating applications, or by separately molding then forming the layers and allowing them to be compressed or heated to mold together. The layers can also be made by a mixture of slot coating the first lubricating material then molding the second lubricating material thereon. Where the lubricating materials form side by side layers as in FIG. 15b, the layers can be formed by co-extrusion via a two orifice die, or they can be formed by having a two orifice die used on a slot coater. Where multiple layers of lubricating materials are used, they can be similar with one or more ingredients, such as a fragrance or colorant or a skin care active such as menthol, being changed. Further, more substantial differences between the first and second layer can also be used. For example, the first layer can comprise an emollient and structuring polymer and the second layer can comprise a further water soluble polymer with a water insoluble polymer.

### IX. Methods of Making

The skin engaging member can be made by a variety of ways. Non-limiting examples of suitable methods of making the skin engaging member include: slot coating the lubricating material into or onto the carrier, dual extrusion of the carrier and the lubricating material, separately molding and/or extruding the carrier and lubricating material then later assembly, and so forth. The skin engaging member may also be made by producing the carrier (e.g. by injection molding), and then compressing the lubricating material into/onto the carrier, e.g. with a ram and with or without the application of heat, or ultrasonically tamping the lubricating material into the carrier, e.g. having poured it into the carrier in powdered form. The skin engaging member may be manufactured by co-extrusion (i.e. via a single extrusion die with two or more orifices so the extrudates merge and weld together as extruded) or two-color molding / two-component molding (i.e. where the carrier or the lubricating material is first molded into a shape which can then be used to mold the other of the carrier or the lubricating material), or by extrusion or molding the carrier and lubricating material separately then assembling in a downstream step in the same process, or in a separate process/location. Suitable examples of methods of making and skin engaging member compositions are provided in U.S. Patent No. 6,298,588, col. 8 - 10. Non-limiting examples of ways to coextrude, or separately mold / extrude then assemble are provided in U.S. Patent Nos. 7,121,754 and 6,298,558. As explained above, the skin engaging member can be deformed to include a non-linear portion before or after the skin care composition is contacted with the carrier.

### X. Methods of Hair Removal (especially shaving)

The hair removal cartridge (especially a razor cartridge) or hair removal device (especially a razor) of the present invention may be used for hair removal (especially shaving), or in a method of hair removal (especially shaving), the method comprising the steps of providing a hair removal cartridge (especially a razor cartridge) or hair removal device (especially a razor) according to the present invention in any form, and passing the same over a surface of the body. Optional additional steps may include wetting the surface, washing the surface, applying one of various commonly known shaving preparations to the surface, (the preceding options typically occurring before passing the hair removal/razor cartridge or hair removal device/razor over the surface), rinsing the surface (which could occur prior to and/or after passing the hair removal/razor cartridge or hair removal device/razor over the surface), drying the surface and applying one of various commonly known post-shave compositions to the surface (the last two steps typically occurring after passing the hair removal/razor cartridge or hair removal device/razor over the surface)

### XI. EXAMPLES

Skin engaging members having a single orifice extending over approximately 75% of the length of the skin-contacting surface (and approximately 90% of the length of exposed blade for shaving) were produced by injection molding a carrier from a blend of polyphenylene oxide and polystyrene (available under the tradename "Noryl"). The lubricating materials in the table below were made by simply mixing the ingredients in accordance with any of the examples provided below and 150mg inserted into the carrier, compacted at 5mm/min to a maximum force of 1500N using a ram with force guage, as available from Instron.

| **Ingredient** | **Comparative Example** | **Inventive Example** |
|---|---|---|
| Polyox Coagulant (PEO)* | 100.00 | 90.00 |
| Pluronic F127 (PEO-PPO Copolymer) | 0.00 | 10.00 |
| Total | 100.00 | 100.00 |

| | | |
|---|---|---|
| * Average molecular weight of 5,000,000 | | |

The coefficient of friction of a common razor cartridge (consistent design between measurements) bearing each of the example skin engaging members (physically identical) above was measured against a polyurethane skin mimic having dimensions of 3 inches (7.5cm) by 6.5 inches (17cm) on a Dia-stron MTT175 instrument fitted with a 2000 g/cm² horizontal load cell. The skin mimic was warmed to a temperature of 27°C, and a downward force of 300g (equivalent as mass) applied. The razor cartridge (including the shaving aid member) was soaked in water at a temperature of 55°C and 1ml of water (also warmed to 55°C) was applied to the skin mimic to wet it prior to measurement. The UvWin software package supplied with the Dia-stron instrument (v.1.24.0000) was used to run three overlapping, subsequent 100mm long strokes with the razor cartridge at a speed of 1500mm/min, ensuring that the razor cartridge did not contact the skin mimic when moving between strokes. Following the five strokes, the skin mimic was removed from the apparatus to be washed with hot water, scrubbed clean by hand, wiped with a paper towel and re-rinsed before being replaced on the apparatus ready for the next sample. The coefficient of friction was calculated for each stroke based upon the middle 70mm length of the stroke (i.e. from 15mm along the stroke to 85mm along the stroke).
This procedure was repeated to yield results from a total of 5 shaving aid members for each example. The results for each of the five strokes (averages of the 5 shaving aid members) appear in the graph below.

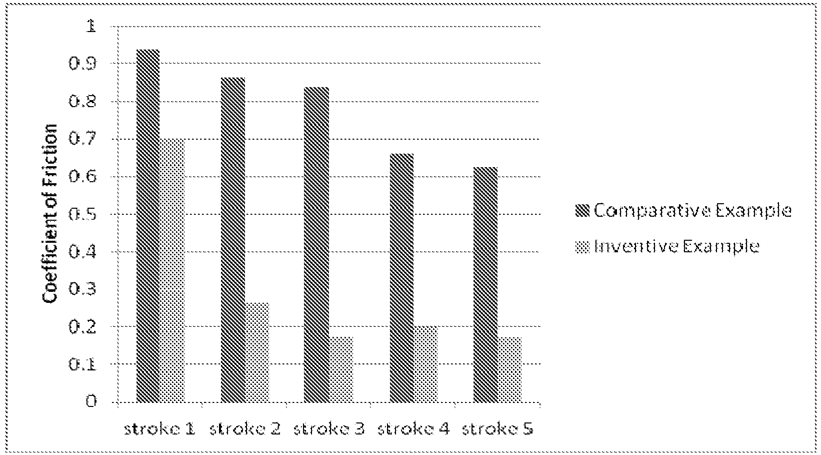

The data clearly shows that the presence of the copolymer of polyethylene oxide and polypropylene oxide reduces the coefficient of friction afforded by the examples tested.

As used herein, molecular weights (mol.wt.s) are provided in unified atomic mass units, daltons, or g/mol.

It should be understood that every maximum numerical limitation given throughout this specification includes every lower numerical limitation, as if such lower numerical limitations were expressly written herein. Every minimum numerical limitation given throughout this specification includes every higher numerical limitation, as if such higher numerical limitations were expressly written herein. Every numerical range given throughout this specification includes every narrower numerical range that falls within such broader numerical range, as if such narrower numerical ranges were all expressly written herein.

All parts, ratios, and percentages herein, in the Specification, Examples, and Claims, are by weight and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A skin engaging member for use on a hair removal device, said skin engaging member comprising:
a. a carrier having a skin contacting surface, said skin contacting surface forming at least one orifice; and
b. a lubricating material releasably engaged with said carrier; said at least one orifice exposing at least a portion of said lubricating material;
wherein the lubricating material comprises a copolymer of polyethylene oxide and polypropylene oxide , wherein said copolymer of polyethylene oxide and polypropylene oxide has an average molecular weight of from 12,000 to 13,000.

2. The skin engaging member of claim 1, wherein the copolymer of polyethylene oxide and polypropylene oxide is a block copolymer, preferably a tri-block copolymer, more preferably a tri-block copolymer having the sequence: polyethylene oxide - polypropylene oxide - polyethylene oxide.

3. The skin engaging member of claim 1, wherein the ratio of polyethylene oxide to polypropylene oxide in the copolymer of polyethylene oxide and polypropylene oxide is in the range of from 1:2.5 to about 1:3, by molecular weight of the copolymer blocks.

4. The skin engaging member of claim 1, wherein the copolymer of polyethylene oxide and polypropylene oxide is from about 5% to about 10% by weight of the lubricating material.

5. The skin engaging member of claim 1, wherein said lubricating material further comprises a water soluble polymer.

6. The skin engaging member of claim 5, wherein the water soluble polymer is selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and mixtures thereof.

7. The skin engaging member of claim 6, wherein the water soluble polymer is polyethylene oxide.

8. The skin engaging member of claim 5, wherein the water soluble polymer has an average molecular weight of from about 300,000 to about 5 million.

9. The skin engaging member of claim 6, wherein the water soluble polymer is present at a level of from about 70% to about 90% by weight of the lubricating material.

10. The skin engaging member of claim 1, wherein said carrier comprises a water insoluble polymer at a level of at least about 35% by weight of said carrier, preferably wherein said water insoluble polymer comprises at least one of: polyethylene, polypropylene, polystyrene, high impact polystyrene, butadiene styrene copolymer, polyacetal, acrylonitrile-butadiene styrene copolymer, ethylene vinyl acetate copolymer, and mixtures thereof.

11. The skin engaging member of claim 1, wherein the lubricating material further comprises a water insoluble polymer, preferably wherein the water insoluble polymer is selected from: polyethylene, polypropylene, polystyrene, high impact polystyrene, butadiene styrene copolymer, polyacetal, acrylonitrile-butadiene styrene copolymer, ethylene vinyl acetate copolymer, and mixtures thereof.

12. The skin engaging member of claim 11 wherein the water insoluble polymer is present at a level of from 1% to about 10% by weight of the lubricating material.

## Patentansprüche

1. Hauteingriffselement zur Verwendung an einer Haarentfernungsvorrichtung, wobei das Hauteingriffselement Folgendes umfasst:
a. einen Träger mit einer hautkontaktierenden Oberfläche, wobei die hautkontaktierende Oberfläche wenigstens eine Öffnung bildet; und
b. ein Gleitmittelmaterial, das lösbar mit dem Träger in Eingriff steht, wobei die wenigstens eine Öffnung wenigstens einen Teil des Gleitmittelmaterials freilegt;
wobei das Gleitmittelmaterial ein Copolymer aus Polyethylenoxid und Polypropylenoxid umfasst, wobei das Copolymer aus Polyethylenoxid und Polypropylenoxid ein durchschnittliches Molekulargewicht von 12.000 bis 13.000 aufweist.

2. Hauteingriffselement nach Anspruch 1, wobei das Copolymer aus Polyethylenoxid und Polypropylenoxid ein Blockcopolymer ist, vorzugsweise ein Triblockcopolymer, mehr bevorzugt ein Triblockcopolymer mit der Sequenz: Polyethylenoxid - Polypropylenoxid - Polyethylenoxid.

3. Hauteingriffselement nach Anspruch 1, wobei das Verhältnis von Polyethylenoxid zu Polypropylenoxid in dem Copolymer aus Polyethylenoxid und Polypropylenoxid bezogen auf das Molekulargewicht der Copolymerblöcke im Bereich von 1:2,5 bis etwa 1:3 liegt.

4. Hauteingriffselement nach Anspruch 1, wobei das Copolymer aus Polyethylenoxid und Polypropylenoxid von etwa 5 Gew.-% bis etwa 10 Gew.-% des Gleitmittelmaterials ausmacht.

5. Hauteingriffselement nach Anspruch 1, wobei das Gleitmittelmaterial ferner ein wasserlösliches Polymer umfasst.

6. Hauteingriffselement nach Anspruch 5, wobei das wasserlösliche Polymer ausgewählt ist aus Polyethylenoxid, Polyvinylpyrrolidon, Polyacrylamid, Polyhydroxymethacrylat, Polyvinylimidazolin, Polyethylenglykol, Polyvinylalkohol, Polyhydroxyethymethacrylat, Silikonpolymeren und Mischungen davon.

7. Hauteingriffselement nach Anspruch 6, wobei es sich bei dem wasserlöslichen Polymer um Polyethylenoxid handelt.

8. Hauteingriffselement nach Anspruch 5, wobei das wasserlösliche Polymer ein durchschnittliches Molekulargewicht von etwa 300.000 bis etwa 5 Millionen aufweist.

9. Hauteingriffselement nach Anspruch 6, wobei das wasserlösliche Polymer in einem Gehalt von etwa 70 Gew.-% bis etwa 90 Gew.-% des Gleitmittelmaterials vorliegt.

10. Hauteingriffselement nach Anspruch 1, wobei der Träger ein wasserunlösliches Polymer in einer Menge von mindestens etwa 35 Gew. -% des Trägers umfasst, wobei das wasserunlösliche Polymer vorzugsweise mindestens eines der Folgenden umfasst: Polyethylen, Polypropylen, Polystyrol, hochschlagfestes Polystyrol, Butadien-Styrol-Copolymer, Polyacetal, AcrylnitrilButadien-Styrol-Copolymer, Ethylen-Vinylacetat-Copolymer und Mischungen davon.

11. Hauteingriffselement nach Anspruch 1, wobei das Gleitmittelmaterial ferner ein wasserunlösliches Polymer umfasst, wobei das wasserunlösliche Polymer vorzugsweise ausgewählt ist aus: Polyethylen, Polypropylen, Polystyrol, hochschlagfestem Polystyrol, Butadien-Styrol-Copolymer, Polyacetal, AcrylnitrilButadien-Styrol-Copolymer, Ethylen-Vinylacetat-Copolymer und Mischungen davon.

12. Hauteingriffselement nach Anspruch 11, wobei das wasserunlösliche Polymer in einem Gehalt von 1 Gew.-% bis etwa 10 Gew.-% des Gleitmittelmaterials vorliegt.

## Revendications

1. Élément de mise en prise de peau pour une utilisation sur un dispositif d'épilation, ledit élément de mise en prise de peau comprenant :
a. un support ayant une surface de contact de peau, ladite surface de contact de peau formant au moins un orifice ; et
b. un matériau lubrifiant mis en prise de manière libérable avec ledit support, ledit au moins un orifice exposant au moins une partie dudit matériau lubrifiant ;
dans lequel le matériau lubrifiant comprend un copolymère d'oxyde de polyéthylène et d'oxyde de polypropylène, dans lequel ledit copolymère d'oxyde de polyéthylène et d'oxyde de polypropylène a une masse moléculaire moyenne allant de 12 000 à 13 000.

2. Élément de mise en prise de peau selon la revendication 1, dans lequel le copolymère d'oxyde de polyéthylène et d'oxyde de polypropylène est un copolymère séquencé, de préférence un copolymère tri-séquencé, plus préférablement un copolymère tri-séquencé ayant la séquence : oxyde de polyéthylène - oxyde de polypropylène - oxyde de polyéthylène.

3. Élément de mise en prise de peau selon la revendication 1, dans lequel le rapport de l'oxyde de polyéthylène à l'oxyde de polypropylène dans le copolymère d'oxyde de polyéthylène et d'oxyde de polypropylène est dans la plage allant de 1:2,5 à environ 1:3, en masse moléculaire des séquences du copolymère.

4. Élément de mise en prise de peau selon la revendication 1, dans lequel le copolymère d'oxyde de polyéthylène et d'oxyde de polypropylène représente d'environ 5 % à environ 10 % en poids du matériau lubrifiant.

5. Élément de mise en prise de peau selon la revendication 1, dans lequel ledit matériau lubrifiant comprend en outre un polymère hydrosoluble.

6. Élément de mise en prise de peau selon la revendication 5, dans lequel le polymère hydrosoluble est choisi parmi l'oxyde de polyéthylène, la polyvinylpyrrolidone, le polyacrylamide, le polyhydroxyméthacrylate, la polyvinyl-imidazoline, le polyéthylène glycol, l'alcool polyvinylique, le polyhydroxyéthylméthacrylate, des polymères de silicone, et leurs mélanges.

7. Élément de mise en prise de peau selon la revendication 6, dans lequel le polymère hydrosoluble est de l'oxyde de polyéthylène.

8. Élément de mise en prise de peau selon la revendication 5, dans lequel le polymère hydrosoluble a une masse moléculaire moyenne d'environ 300 000 à environ 5 millions.

9. Élément de mise en prise de peau selon la revendication 6, dans lequel le polymère hydrosoluble est présent à un taux allant d'environ 70 % à environ 90 % en poids du matériau lubrifiant.

10. Élément de mise en prise de peau selon la revendication 1, dans lequel ledit support comprend un polymère insoluble dans l'eau à un taux d'au moins 35 % en poids dudit support, de préférence dans lequel ledit polymère insoluble dans l'eau comprend au moins l'un parmi : du polyéthylène, du polypropylène, du polystyrène, du polystyrène à haut impact, un copolymère butadiène-styrène, du polyacétal, un copolymère acrylonitrile-butadiène styrène, un copolymère éthylène-acétate de vinyle et des mélanges de ceux-ci.

11. Élément de mise en prise de peau selon la revendication 1, dans lequel le matériau lubrifiant comprend en outre un polymère insoluble dans l'eau, de préférence dans lequel le polymère insoluble dans l'eau est choisi parmi : du polyéthylène, du polypropylène, du polystyrène, du polystyrène à haut impact, un copolymère butadiène-styrène, du polyacétal, un copolymère acrylonitrile-butadiène styrène, un copolymère éthylène-acétate de vinyle, et des mélanges de ceux-ci.

12. Élément de mise en prise de peau selon la revendication 11, dans lequel le polymère insoluble dans l'eau est présent à un taux allant de 1 % à environ 10 % en poids du matériau lubrifiant.
